**Europäisches Patentamt**

**European Patent Office**

**Office européen des brevets**

(11) Veröffentlichungsnummer: **0 152 722**
**A1**

## (12) EUROPÄISCHE PATENTANMELDUNG

(21) Anmeldenummer: **84810640.7**

(22) Anmeldetag: **19.12.84**

(51) Int. Cl.⁴: **A 61 L 9/12**
**// A47K10/16, A47K10/32**

(30) Priorität: **20.12.83 CH 6773/83**

(43) Veröffentlichungstag der Anmeldung: **28.08.85**
**Patentblatt 85/35**

(84) Benannte Vertragsstaaten: **AT BE CH DE FR GB IT LI NL SE**

(71) Anmelder: **SIPURO AG, Erlenauweg 13, CH-3110 Münsingen (CH)**

(72) Erfinder: **Rohrer, Hermann, Dr., Erlenauweg 13, CH-3110 Münsingen (CH)**

(74) Vertreter: **Ritscher, Thomas, Dr. et al, RITSCHER & SEIFERT Auf der Mauer 4, CH-8001 Zürich (CH)**

(54) **Vorrichtung zum Desodorieren von Räumen.**

㉗ Die Vorrichtung dient zum geruchsüberdeckenden Desodorieren von Räumen, insbesondere WC-Räumen oder/und Badezimmern, in welchen sich mindestens ein Dispenser (10) für eine auswechselbare Hygienepapierrolle (11) mit einer im Inneren der Rolle liegenden Wickelhülse (111) befindet; der Dispenser (10) hat eine Halterung (14) zur abrollbaren Führung der Wickelhülse (111) der Rolle (11); ein praktisch hohlzylindrischer Körper (15), der Duftstoff sowie Trägermaterial für den Duftstoff enthält, ist zum Einschieben zwischen die Wickelhülse (111) und die Halterung (14) bestimmt; das Trägermaterial besteht aus Kunststoffmasse, und der Duftstoff ist mindestens zum Teil in der Kunststoffmasse praktisch molekulardispers verteilt.

0152722

## Vorrichtung zum Desodorieren von Räumen

Die Erfindung betrifft eine Vorrichtung zum geruchsüberdekkenden Desodorieren von Räumen, insbesondere WC- oder/und Badezimmerräumen.

Zum Desodorieren der Raumluft sind verschiedene Methoden bekannt, die sich im wesentlichen dadurch unterscheiden, ob der störende Geruch aus bzw. mit der Raumluft entfernt wird, z. B. durch Luftaustausch und/oder Geruchsadsorption bzw. -vernichtung, oder ob man den störenden Geruch mindestens teilweise mit Duftstoff überdeckt; die letztere Methode, d.h. die geruchsüberdeckende Raumdesodorierung, ist dabei vergleichsweise am einfachsten bzw. preiswertesten und kann entweder als einzige Desodoriermethode oder als zusätzliche Massnahme, z. B. zusammen mit Luftaustausch, eingesetzt werden. Allgemein werden hierfür Duftstoffe synthetischer oder natürlicher Herkunft verwendet, beispielsweise Parfumöle bzw. Parfumkompositionen, die von den Geruchsorganen leichter bzw. stärker wahrgenommen werden, als die unter normalen Umständen beispielsweise im Haushalt auftretenden und als unangenehm empfundenen Gerüche.

Im allgemeinen ist zum geruchsüberdeckenden Desodorieren eine Verteilung des Duftstoffes in der Raumluft erforderlich. Beim permanenten Desodorieren geschieht dies allgemein durch Verdampfen des Geruchsverbesserungsmittels, während eine temporäre geruchsüberdeckende Wirkung meist durch Versprühen, etwa aus Aerosoldosen, erzielt wird.

In WC-Räumen bzw. Badezimmern mit WC-Schüsseln ist die permanente geruchsüberdeckende Desodorierung auch zusätzlich zur Belüftung bzw. Luftzirkulation üblich. Die dazu bekannten Vorrichtungen enthalten z.B. ein mit Duftstoff imprägniertes Faservlies, das an einem Träger oder Rahmen befestigt ist und mit einer Halterung z. B. auf eine Abstellfläche gestellt oder an der Wand des WC-Raumes angebracht werden kann; weder die eine noch die andere Art der Anbringung ist optimal; ferner ist die Duftstoffkapazität der bekannten Vorrichtungen für diese Verwendung relativ begrenzt und die Duftstoffabgabe entweder im wesentlichen gleichbleibend oder konstant abnehmend.

Aufgabe der Erfindung ist eine besonders für WC-Räume oder/ und Badezimmer mit WC-Schüsseln geeignete Desodoriervorrichtung, die keine besondere Halterung und keinen freien Platz zum Aufhängen bzw. Aufstellen benötigt, eine vergleichsweise höhere Duftstoffabgabekapazität besitzt, eine benützungsabhängige und vorübergehend verstärkte Duftstoffabgabe ohne besondere bzw. kostspielige Hilfsmassnahmen ermöglicht, einfach aufgebaut ist, einen zuverlässigen Betrieb bietet und kostengünstig hergestellt werden kann.

Diese Aufgabe wird erfindungsgemäss gelöst durch eine Vorrichtung zum geruchsüberdeckenden Desodorieren von Räumen, insbesondere WC- oder/und Badezimmerräumen, in welchen sich mindestens ein üblicher Dispenser, z. B. Klopapierhalter, für eine auswechselbare Hygienepapierrolle üblicher Art, z. B. eine Klopapierrolle, mit einer im Inneren der Rolle liegenden Wikkelhülse, z. B. aus Karton oder Papierlagen, befindet, welcher Dispenser eine übliche Halterung zur abrollbaren Führung der Wickelhülse der Rolle besitzt; im allgemeinen haben solche Dispenser keine besonderen Gehäuse.

Als Halterungen sind dabei solche Einrichtungen geeignet, welche in mindestens eines der beiden offenen Enden der Wickelhülse eingreifen und sich über mindestens einen Teil der axialen Länge der Wickelhülse erstrecken; Beispiele geeigneter Halterungen sind einfache Bügel, auf welche die Wickelhülsen aufgeschoben werden können, mit oder ohne besondere Führungsrolle, sowie seitliche Bügelpaare und längliche Rollen, die an beiden Enden mit Stiften oder Schrauben in entsprechenden Backen drehbar gelagert sind, oder nicht-drehbare und mit einem Ende an einer Wandplatte befestigte Führungszylinder, deren Aussendurchmesser meist um 10 bis 20 mm kleiner ist, als der Innendurchmesser der üblichen Wickelhülsen von Hygienepapierrollen.

Im allgemeinen liegt die Längsachse des sich in die Wickelhülse erstreckenden Teils des Dispensers nicht koaxial mit der Wickelhülse, d. h. die Wickelhülse liegt nur mit einem jeweils oben liegenden Innenflächenbereich auf der Halterung.

Die erfindungsgemässe Vorrichtung ist gekennzeichnet durch einen praktisch hohlzylindrischen Körper, der Duftstoff sowie Trägermaterial für den Duftstoff enthält und zum Einschieben zwischen die Wickelhülse der Rolle und die Halterung bestimmt ist, wobei das Trägermaterial aus Kunststoffmasse besteht und der Duftstoff mindestens teilweise in der Kunststoffmasse "gelöst", d. h. praktisch molekulardispers darin verteilt ist.

Der Körper, im folgenden auch Desodorierkörper genannt, ist praktisch hohlzylindrisch in dem Sinne, dass eine allfällige Konizität - die aus fertigungstechnischen Gründen wünschbar sein kann - nicht so ausgeprägt ist, dass dadurch das Einschieben zwischen die Wickelhülse und die Halterung merklich erschwert wird. Ferner bedeutet die Bezeichnung "praktisch hohlzylindrisch", dass auf der Innenfläche oder/und Aussenfläche des Körpers Rillen oder/und Vorsprünge vorhanden sein können, soweit diese das Anbringen des Körpers zwischen der Wickelhülse der Hygienepapierrolle und der Halterung des Dispensers nicht beeinträchtigen.

Gemäss einer allgemein bevorzugten Ausführungsform der Erfindung ist der Körper ein integrales, d. h. einstückiges, praktisch rohr- oder hülsenförmiges Gebilde.

Vorzugsweise sind die Abmessungen des Desodorierkörpers so bemessen, dass dieser bequem in die Wickelhülse der üblichen Hygienepapierrollen bzw. Klopapierrollen eingeschoben werden kann, und bequem in den Zwischenraum zwischen Wickelhülse und Halterung passt; die axiale Länge des Desodorierkörpers ist zweckmässig höchstens ebenso gross, wie die axiale Länge der Wickelhülse; es können aber auch vergleichsweise kurze Desodorierkörper verwendet werden, d. h. solche, die mehr als Ringe denn als Hülsen zu bezeichnen sind.

Für die weitaus meisten Anwendungen sind annähernd rohrförmige Desodorierkörper mit folgenden Abmessungen geeignet: Wandstärke 0,5 bis 5 mm, vorzugsweise 1 bis 4 mm; Aussendurchmesser 20 bis 50 mm, vorzugsweise 30 bis 40 mm; Länge 20 bis 300 mm, vorzugsweise 70 bis 120 mm.

Um eine allenfalls notwendige oder wünschbare Anpassung des hohlzylindrischen Körpers vor dem Einsetzen in die Wickelhülse bzw. vor dem Aufschieben auf die Halterung zu bewirken, kann der Körper eine praktisch über seine axiale Länge sich erstreckende Trennstelle, z. B. eine gerade, gekrümmte oder anders geformte Durchschneidung bzw. eine entsprechende, z.B. von Hand aufreissbare Solltrennstelle besitzen; da der hohlzylindrische Körper aus den weiter unten erläuterten Gründen meist flexibel und relativ weich ist, kann er im Bedarfsfall auch mit einer normalen Schere in Längsrichtung aufgeschnitten werden, wenn dies zum Einsetzen nötig oder wünschbar ist. In ähnlicher Weise kann nötigenfalls auch die Länge des Körpers verändert werden, und zwar einschliesslich einer Verkürzung, z. B. zur Anpassung an die Wickelhülse oder/und zur Verminderung der Duftstofffreigabe.

Die als Trägermaterial für den Duftstoff bzw. eine Duftstoffmischung verwendete Kunststoffmasse muss befähigt sein, den Duftstoff mindestens teilweise zu "lösen", worunter hier allgemein eine praktisch molekulardisperse Verteilung des Duftstoffes in der Kunststoffmasse verstanden wird, wie sie z.B. für flüssige Weichmacher bekannt ist und in Abhängigkeit von der Menge des Duftstoffes eine mehr oder weniger ausgeprägte und durchaus vorteilhafte Erweichung oder Plastifizierung bzw. Quellung der Kunststoffmasse zur Folge hat.

0152722

Es wurde gefunden, dass typische, d. h. organische und allgemein oleophile bzw. hydrophobe Duftstoffe der in der Desodoriertechnik bzw. technischen Parfümerie üblichen Art in meist mehr oder weniger thermoplastischen und jedenfalls nicht lösungsmittelfesten Kunststoffmassen bei erhöhten und normalen Temperaturen in erheblichen Anteilen, z. B. bis etwa 50 % des Gewichtes der Mischung, löslich sind, ohne dass die Kunststoffmasse dadurch ihren Zusammenhalt verliert.

Eine begleitende ausgeprägte Weichmacherwirkung des Duftstoffes auf die Kunststoffmasse - d.h. Beibehaltung eines molekulardispersen Zustandes, der z. B. durch Erwärmen auf Schmelztemperaturen oder/und Verkneten zustandegekommen ist, auch bei Normaltemperatur und längerem Lagern - ist dabei allgemein vorteilhaft für die Erfindung, weil der Desodorierkörper zwar noch fest im Sinne von zusammenhängend aber vorzugsweise auch gegen Abrieb verhältnismässig labil sein soll.

Ein Desodorierkörper aus einer zwar abrieblabilen aber selbsttragenden Masse hat den zusätzlichen Vorteil, dass bei bestimmungsgemässer Verwendung die bei Betätigung des Dispensers, d. h. beim Abziehen von Papierabschnitten von der Rolle, normalerweise zwischen der Wickelhülse und der Halterung auftretenden Reibungs- oder Scherkräfte verhältnismässig geringe Duftstoffanteile abreiben und dadurch eine vorübergehend etwas verstärkte Duftstoffabgabe bewirken.

Ein Duftstoffanteil wird hier dann als "verhältnismässig gering" angesehen, wenn er im Verhältnis zur Masse des Körpers sehr klein ist und weniger als 1 Promille beträgt, aber organoleptisch signifikant, d. h. deutlich wahrnehmbar ist.

Im allgemeinen besteht der Desodorierkörper aus einer Mischung von 45 bis 95 Gew.%, vorzugsweise 50 bis 75 Gew.%, Kunststoffmasse, 5 bis 40 Gew.%, vorzugsweise 20 bis 30 Gew.%, Duftstoff und 0 bis 50 Gew.%, vorzugsweise 5 bis 20 Gew.%, Hilfsstoff, der mindestens zum Teil und vorzugsweise überwiegend ein feinteiliger (Teilchengrösse typisch unter 1 mm, insbesondere unter 100 µm) fester Zusatzstoff ist; der Hilfsstoff kann geringe Farbstoff- oder Pigmentanteile von typisch weniger als 1 Gew.%, insbesondere unter 0,1 Gew.%, bezogen auf die gesamte Mischung, enthalten.

Die Kunststoffmasse kann meist ganz oder teilweise aus thermoplastischen Polymeren, z. B. Copolymeren aus Ethylen und Vinylacetet (EVA), wie sie von der Firma DuPont unter der Marke ELVAX (z. B. Typen 150-360) erhältlich sind, technischen Homo- oder Copolymeren von Vinylchlorid (PVC) und thermoplastischen Elastomeren aus Copolymeren oder Polymermischungen von Styrol und Butadien, wie sie z. B. von der Firma Shell unter der Marke CARIFLEX erhältlich sind, bestehen.

Andere Polymere, wie Polyethylen, Copolymere aus Acrylnitril, Butadien und Styrol, sind geeignet, sofern sie mit dem verwendeten Duftstoff bzw. Duftstoffgemisch Lösungen bzw. Molekulardispersionen bilden können.

Die Kunststoffmasse kann ferner auch aus Mischungen der oben genannten thermoplastischen Polymeren miteinander oder/und mit kompatiblen wachs- oder paraffinartigen Zusätzen bestehen.

Als Duftstoffe kommen die üblichen organischen, meist oleophilen technischen Parfums bzw. Parfumkompositionen in Betracht, die bei Raumtemperatur und molekulardisperser Verteilung in der Kunststoffmasse eine für die Desodorierung ausreichende Verflüchtigungsrate besitzen; Terpene bzw. Terpenoide sind dabei weniger bevorzugt, soweit sie zur Entmischung ("Ausschwitzen", d. h. Bildung öliger Einschlüsse oder Oberflächenschichten) neigen.

Die Auswahl geeigneter Duftstoffe bzw. Duftstoffgemische, die sich in der jeweils gegebenen Kunststoffmasse praktisch molekular dispergieren lassen und diesen Zustand unter Normalbedingungen im wesentlichen beibehalten, ist eine fachmännische Massnahme. Nötigenfalls kann man die Eignung anhand eines einfachen Versuches kontrollieren, indem man aus dem Kunststoff und dem Duftstoff, gegebenenfalls mit Zusatzstoff, in der Wärme eine möglichst homogene Mischung bildet und die Gleichmässigkeit der Mischung nach dem Abkühlen bzw. die Stabilität der Mischung unter den normalen Lagerungs- und Verwendungsbedingungen prüft.

Vorzugsweise enthält die Masse als Hilfsstoff mindestens einen feinteiligen, inerten, festen und z. B. mineralischen Zusatzstoff, wie $SiO_2$ (z. B. als gefällte Kieselsäure, als Kieselgur oder als pyrogenes $SiO_2$), $Al_2O_3$, Steinmehl, Gips, Glaspulver und dergleichen Fest- bzw. Füllstoffe mit mehr oder weniger organosorptivem Charakter, in homogener und/oder heterogener (z. B. inselartiger) Verteilung.

Dies ermöglicht die Bildung vorteilhafter Ausgangsmischungen einfach dadurch, dass man Kunststoffgranulat mit Duftstoff tränkt und dann mit genügend festem Zusatzstoff vermischt, um eine noch rieselfähige Mischung zu bilden; diese kann dann di-

rekt als Speisung der Formmaschine, wie Spritzgussmaschine oder Strangpresse, verwendet werden, in der die Mischung unter Erwärmung plastifiziert und als plastische Masse geformt wird.

In typischen Anteilen von 5 bis 50 %, insbesondere 10 bis 40 %, des Gewichtes der fertigen Masse bieten solche Zusatzstoffe weitere Vorteile, indem sie eine allfällige Neigung des Duftstoffes zur Entmischung ("Ausschwitzen") kompensieren, eine erhöhte bzw. verbesserte Duftstoffaufnahme oder -verteilung, eine gefällige Strukturierung der Masse und eine Regelung der Abriebslabilität bieten.

Wenn mindestens ein Teil des Feststoffes heterogen, z. B. in Form von inselartigen Ballungen oder streifenartigen Häufungen, in transparenter bzw. transluzenter, farbloser oder gefärbter Masse verteilt ist, bietet dies ein gefälliges strukturiertes Aussehen des Desodorierkörpers und gegebenenfalls eine Vergrösserung der Duftstoff abgebenden Oberfläche.

- 9 -                                          0152722

Die Erfindung wird anhand der Zeichnung in Beispielen weiter
erläutert. Es zeigen:

Fig. 1   die Seitenansicht eines erfindungsgemässen Formkörpers bei Verwendung in einem üblichen Toilettenpapier-Dispenser mit aufgeschobener Papierrolle,

Fig. 2   die perspektivische Darstellung des in Fig. 1 ge-
         zeigten Toilettenpapier-Dispensers beim Aufschie-
         ben der Papierrolle,

Fig. 3   die perspektivische Darstellung einer bevorzugten
         Ausführungsform des Formkörpers,

Fig. 4   die perspektivische Darstellung eines längsgerippten Formkörpers,

Fig. 5   die teilweise geschnittene Darstellung eines spiralgerippten Formkörpers,

Fig. 6   die Seitenansicht eines in Längsrichtung zertrennten Formkörpers, und

Fig. 7   die Seitenansicht eines Formkörpers mit überlappenden Längstrennflächen.

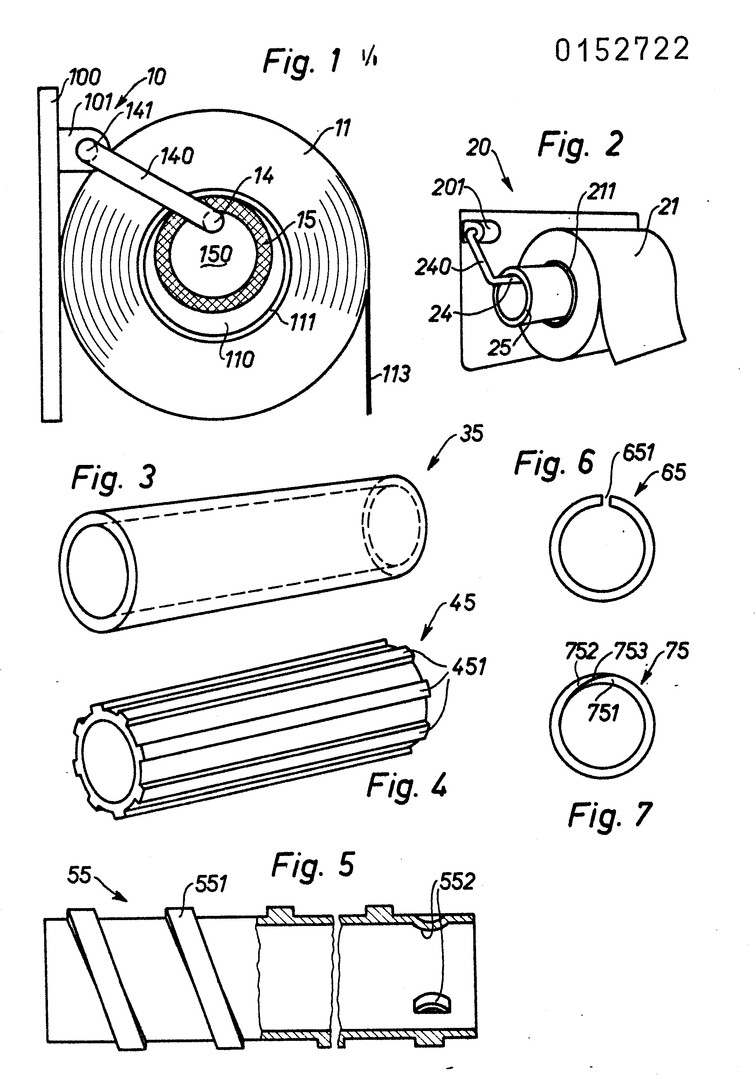

Im einzelnen zeigt Fig. 1 einen Dispenser 10 mit einer Befestigungsplatte 100, an der in einer Oese 101 ein annähernd
U-förmiger Bügel 140 schwenkbar um seinen oberen Schenkel 141
eingesetzt ist. Der untere Schenkel des Bügels bildet die
Rollenhalterung 14, die sich in den Hohlraum 110 der Toilettenpapierrolle 11 hinein und in axialer Richtung durch diesen hindurch erstreckt.

Zwischen die Wickelhülse 111 der Rolle 11 und die Rollenhalterung 14 ist der hohlzylindrischen Körper 15 eingeschoben,
der (in nicht dargestellter Weise) eine praktisch der axialen Länge der Rolle 11 bzw. der Wickelhülse 111 entsprechende Länge und einen ringförmigen Querschnitt besitzt.

Der hohlzylindrische Körper 15 ist ein durch Spritzgiessen hergestellter, annähernd rohrförmiger Formkörper mit einem Aussendurchmesser von 35 mm, einer Wandstärke von 2 mm und einer Länge von 90 mm aus einer praktisch transparenten flexiblen Masse, die aus etwa 36 Gew.% oleophilem Parfum als Duftstoff, 10 Gew.% $SiO_2$ als gefällte Kieselsäure, etwa 0,1 Gew.% blauem Farbstoff und Copolymer aus Ethylen und Vinylacetat (ELVAX) als Rest besteht.

Im Ruhezustand kann Raumluft durch den zylindrischen Innenraum 110 der Toilettenpapierrolle 11 sowie durch den Innenraum 150 des Formkörpers 15 hindurchströmen, was eine kontinuierliche und wegen der relativ grossen Duftstoffmasse anhaltende Desodorierung bietet.

Zur Betätigung des Dispensers 10 werden in üblicher Weise Papierabschnitte am freien Ende 113 der Rolle abgezogen und abgerissen, wodurch die Innenfläche der Wickelhülse 111 reibend über den anliegenden Teil der Oberfläche des Formkörpers 15 gleitet und dessen Innenfläche seinerseits gegen den anliegenden Teil der Halterung 14 reibt.

Wenn der Formkörper 15 aus abrieblabiler Masse besteht, wird an dessen Oberfläche, insbesondere im Bereich des Kontaktes mit der Halterung 14, zusätzlicher Duftstoff durch Abrieb freigesetzt, was eine verstärkte Duftstoffabgabe bewirkt.

Fig. 2 zeigt in perspektivischer vereinfachter Darstellung den Dispenser 20, dessen Rollenbügel 240 mit einem Ende in der Oese 201 drehbar geführt ist und dessen anderes Ende sich als Halterung 24 in die Wickelhülse 211 der Papierrolle 21 erstreckt.

In der dargestellten Position ist der Formkörper 25 bereits weitgehend auf die Halterung 24 aufgeschoben, während die Papierrolle 21 erst zu einem Teil auf den Formkörper 25 geschoben ist. In der (nicht dargestellten) Endstellung liegen die Rolle 21 und der Formkörper 25 praktisch am Bügel 240 an.

Fig. 3 zeigt einen zur Verwendung gemäss den Fig. 1 und 2 geeigneten Formkörper 35.

Gemäss Fig. 4 kann ein ähnlicher Formkörper 45 aussenseitig mit mehreren parallelen Längsrippen 451 versehen sein; dadurch kann die Durchströmung und der Abrieb des Formkörpers verstärkt werden.

Der in Fig. 5 teilweise im Schnitt dargestellte Formkörper 55 hat aussenseitig einen spiralförmigen Wulst 551 und gewünschtenfalls eine innenseitige Auswölbung 552, die zur Verkeilung mit einer entsprechend dicken zylindrischen Halterung (nicht dargestellt) dient und den Formkörper beim Rollenwechsel am Dispenser hält.

Die Fig. 6 und 7 zeigen die Formkörper 65 und 75 zur schematischen Darstellung einer linearen und aufgeweiteten Trennstelle 651 bzw. einer aus einander überlappenden Enden 751, 752 bestehenden und noch nicht aufgeweiteten Trennstelle 753.

Im Rahmen der Erfindung liegen zahlreiche Aenderungen; so können beispielsweise Hygienepapierrollen verwendet werden, die keine besonderen Wickelkerne besitzen, sondern mit der innersten Papierlage direkt auf einem erfindungsgemässen Formkörper liegen, der auch selbst als Wickelkern dienen kann. Dadurch kann die auch mit Wickelkernen aus Pappe bereits deutliche und vorteilhafte Parfümierung des Hygienepapiers verstärkt werden.

Obwohl die Erfindung für Toilettendesodorierung besonders wichtig ist, kann sie auch für andere Arten von Hygienepapierrollen, z. B. in Form von Einmalhandtüchern für Wasch- und Badezimmer, oder für Küchen dienen. Die beschriebenen Formkörper können aber nicht nur im Haushalt, sondern mit Vorteil auch in gewerblichen Räumen oder in öffentlich zugänglichen Toiletten, Waschräumen und dergleichen erfindungsgemäss eingesetzt werden.

Patentansprüche

1. Vorrichtung zum geruchsüberdeckenden Desodorieren von Räumen, insbesondere WC-Räumen oder/und Badezimmern, in welchen sich mindestens ein Dispenser (10) für eine auswechselbare Hygienepapierrolle (11) mit einer im Inneren der Rolle liegenden Wickelhülse (111) befindet, welcher Dispenser (10) eine Halterung (14) zur abrollbaren Führung der Wickelhülse (111) der Rolle (11) besitzt, gekennzeichnet durch einen praktisch hohlzylindrischen Körper (15), der Duftstoff sowie Trägermaterial für den Duftstoff enthält und zum Einschieben zwischen die Wickelhülse (111) der Rolle (11) und die Halterung (14) bestimmt ist, wobei das Trägermaterial aus Kunststoffmasse besteht und der Duftstoff mindestens zum Teil in der Kunststoffmasse praktisch molekulardispers verteilt ist.

2. Vorrichtung nach Anspruch 1, dadurch gekennzeichnet, dass der Körper (15) ein Formkörper aus einer abrieblabilen flexiblen Masse ist, welche im wesentlichen aus thermoplastischem Kunststoff mit darin gelöstem Duftstoff sowie gegebenenfalls einem feinteiligen mineralischen Zusatz besteht, um durch die bei Betätigung des Dispensers (10) auftretenden Kräfte zwischen der Wickelhülse (111) und der Halterung (14) verhältnismässig geringen Duftstoffanteile abzureiben und dadurch eine vorübergehend verstärkte Duftstoffabgabe zu bewirken.

3. Vorrichtung nach einem der Ansprüche 1 oder 2, dadurch gekennzeichnet, dass der Körper (15) aus einer Mischung von 45 bis 95 Gew.% Kunststoffmasse, 5 bis 40 Gew.% Duftstoff und 0 bis 50 Gew.% Hilfsstoff besteht, welcher Hilfsstoff mindestens zum Teil ein fester feinteiliger Zusatzstoff ist.

4. Vorrichtung nach Anspruch 2 oder 3, dadurch gekennzeichnet, dass der feste Zusatzstoff teilweise inhomogen in der Masse verteilt ist.

5. Vorrichtung nach einem der Ansprüche 1 bis 4, dadurch gekennzeichnet, dass der hohlzylindrische Körper (15) eine Wandstärke von mindestens 0,5 mm und höchstens 5 mm, sowie einen Aussendurchmesser von mindestens 20 mm und höchstens 50 mm und eine Länge von mindestens 20 mm und höchstens 300 mm, vorzugsweise höchstens 120 mm, hat.

6. Vorrichtung nach einem der Ansprüche 1 bis 5, dadurch gekennzeichnet, dass der Mantel des hohlzylindrischen Körpers (15) eine praktisch über die Länge des Körpers sich erstreckende Trennstelle oder Soll-Trennstelle besitzt, um den Aussen- oder/und Innendurchmesser des Körpers (15) vor dem Einschieben zwischen der Wickelhülse (111) und der Halterung (14) zur Anpassung zu verändern.

7. Vorrichtung nach einem der Ansprüche 1 bis 6, dadurch gekennzeichnet, dass die Kunststoffmasse mindestens überwiegend ein Copolymer aus Ethylen und Vinylacetat ist.

Fig. 1  ¼

Fig. 2

Fig. 3

Fig. 6

Fig. 4

Fig. 7

Fig. 5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-2 988 283 (I.W. GARFIELD) <br> * Ansprüche 1-4; Figuren 1,3,4 * | 1,2,5 | A 61 L 9/12 // <br> A 47 K 10/16 <br> A 47 K 10/32 |
| Y | | 3-4,7 | |
| | --- | | |
| X | US-A-2 728 604 (I.W. GARFIELD) <br> * Ansprüche 1-10; Figur 3 * | 1,2,5 | |
| Y | | 3-4,7 | |
| | --- | | |
| Y | US-A-4 095 031 (E.J. ENGLE) <br> * Spalte 2, Zeilen 46-49; Beispiel 4 * | 3-4,7 | |
| | --- | | RECHERCHIERTE SACHGEBIETE (Int. Cl.4) |
| A | DE-A-1 130 559 (B. VENEZIAN) <br> * Ansprüche 1-6 * | 1 | |
| | --- | | |
| A | FR-A-1 409 444 (M. COUTURIER) | 1 | A 61 L <br> A 47 K |
| | ----- | | |

Der vorliegende Recherchenbericht wurde für alle Patentansprüche erstellt.

| Recherchenort <br> DEN HAAG | Abschlußdatum der Recherche <br> 12-04-1985 | Prüfer <br> PELTRE CHR. |
|---|---|---|